# EUROPEAN PATENT APPLICATION

(11) **EP 4 844 775 A1**
(43) Date of publication of application: **25.11.2026**
(21) Application number: 25178053.2
(22) Date of filing: 21.05.2025
(51) Int. Cl.: G16H 15/00, G08B 25/00, G16H 40/20, G16H 40/63, G16H 50/20, G16H 50/70

(54) **DEVICE, SYSTEM AND METHOD FOR IMPROVED PRIORITIZATION OF PATIENT-RELATED MEDICAL ALARMS**

(71) Applicant: Koninklijke Philips N.V., 5656 AE Eindhoven (NL)
(72) Inventor: BIST, Cambodge, 5656 AG Eindhoven (NL)
(74) Representative: Philips Intellectual Property & Standards

(57) **Abstract**

The present invention relates to a device, system and method for improved prioritization of patient-related medical alarms. The device comprises an input unit configured to obtain real-time medical device data obtained in real-time from at least one medical device and historical medical data of the patient, the real-time medical device data relating to any one of monitoring of the patient and a malfunction of a medical device used for monitoring or treatment of the patient; a processing unit configured to identify a triggering feature, being a feature of the real-time medical device data indicative of an event which triggers an alarm; to determine, based on the real-time medical device data and the historical medical data a priority level indicating a priority of the alarm; and to identify at least one contributing feature, being a feature of the real-time medical device data or the historical medical data different from the triggering feature, contributing to the determination of the priority level; and an output unit configured to provide alarm priority data comprising
information about the identified triggering feature, the determined priority level, and the at least one identified contributing feature.

## Description

### FIELD OF THE INVENTION

The present invention relates to a device, system and method for improved prioritization of patient-related medical alarms.

### BACKGROUND OF THE INVENTION

Alarm management plays an important role in hospitals for patient safety and optimizing clinical workflows. Alarms inform clinicians about abnormal physiological parameters (vital signs) of patients (e.g. SpO2, ECG, NIBP) or technical issues with a monitoring device or a treatment device. However, too many alarms overwhelm clinicians and create "alarm fatigue". There are many reasons why this happens: too many alarms (clinicians cannot prioritize their task); too many false alarms (clinicians get desensitized to alarms); unclear alarms (clinicians cannot decipher cryptic text of alarms); high workload (clinicians are overburdened and ignore alarms) etc.

In order to understand the underlying problem, it is necessary to understand how alarms are classified. Alarm standards ask manufacturers of medical devices to assign alarm priorities based on two axes:
- safety: death or irreversible injury, reversible injury, and discomfort or reversible injury, and
- response time: immediate, prompt or delayed.

However, the definition of response time (immediate, prompt or delayed) is unclear. This makes it more difficult to assign alarm priorities to individual alarms and often leads to higher alarm prioritization to ensure safety. Furthermore, clinicians struggle with assigning a priority without complete clinical context, resulting in assigning a higher priority to an alarm to be safer. Accordingly, the end user is impacted especially if the alarming situation is incorrectly prioritized.

Typically, in an alarming system for individual alarms the system announces the alarm as per defined alarm assessment process by the manufacturer. In case multiple alarms are occurring at the same time, the priority of the highest alarm is announced. When there is an individual or multiple alarming situation the end user needs to make a decision based on the active alarm and patient vital data (current and past). However, particularly for the reasons given above, it is common that alarm priority shown by medical systems does not reflect the actual priority of situation. Furthermore, responding to alarm is left completely to the decision of the clinician. This can introduce some inefficiencies and lack of standardization. Responding to alarms based on interpretation of the priorities from existing alarm systems are dependent on the culture of the hospital and their workflow. Therefore, some hospitals are considered to be less "safe" than others.

### SUMMARY OF THE INVENTION

It is an object of the present invention to improve alarm classification/prioritization. In particular, it is an object to reduce alarm fatigue, particularly by reducing the number of alarms and more particularly by reducing the number of false alarms.

In a first aspect of the present invention a device for improved prioritization of patient-related medical alarms is presented, the device comprising:
- an input unit configured to obtain real-time medical device data obtained in real-time from at least one medical device and historical medical data of the patient, the real-time medical device data relating to any one of monitoring of the patient and a malfunction of a medical device used for monitoring or treatment of the patient;
- a processing unit configured to:
   - identify a triggering feature, being a feature of the real-time medical device data indicative of an event which triggers an alarm;
   - determine, based on the real-time medical device data and the historical medical data a priority level indicating priority of the alarm; and
   - identify at least one contributing feature, being a feature of the real-time medical device data and/or the historical medical data different from the triggering feature, contributing to the determination of the priority level; and
- an output unit configured to provide alarm priority data comprising information about the identified triggering feature, the determined priority level, and the at least one identified contributing feature.

In a second aspect of the present invention a system for improved prioritization of patient-related medical alarms is presented, the system comprising:
- a device as presented herein; and
- a rendering unit configured to render visual and/or audible information in accordance with the alarm priority data.

In a third aspect of the present invention a method for improved prioritization of patient-related medical alarms is presented, the method comprising:
- obtaining real-time medical device data obtained in real-time from at least one medical device and historical medical data of the patient, the real-time medical device data relating to any one of monitoring of the patient and a malfunction of a medical device used for monitoring or treatment of the patient;
- identifying a triggering feature, being a feature of the real-time medical device data indicative of an event which triggers an alarm;
- determining, based on the real-time medical device data a priority level indicating a priority of the alarm;
- identifying at least one contributing feature, being a feature of the real-time medical device data and/or the historical medical data different from the triggering feature, contributing to the determination of the priority level; and
- providing alarm priority data comprising information about the identified triggering feature, the determined priority level and the at least one identified contributing feature.

In yet a further aspect of the present invention, there is provided a corresponding computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, computer program and medium have similar and/or identical preferred embodiments as the claimed device and system, in particular as defined in the dependent claims and as disclosed herein.

The present invention is based on the idea to provide an alarm with contextual information for a given alarming situation to caregivers.

The input unit of the device is configured to obtain real-time medical device data obtained in real-time particularly from one or more of a patient monitor, a patient treatment device such as a ventilator or an infusion pump, for example. Real-time medical device data may comprise vital signs data of the patient, medication data, treatment data or other data comprising information about the patient, which may be obtained in real-time from at least one medical device. Said data may also be stored in the device for later use as historical device data. Similarly, these data may be transmitted to a database/storage for later use.

The historical medical data may be obtained from a database/storage such as an electronic health record system of a hospital. The historical medical data may comprise information about one or more of (prior/historical) alarms, vital signs, medication dosage, medication type, start and/or end time of medication/treatment etc. of the patient.

Based on the real-time medical device data the processing unit is configured to identify a triggering feature, i.e. a feature which is indicative of an event which triggers an alarm, and to determine a priority level indicating the priority of the alarm. In this respect the processing unit may comprise an "alarm priority level classifier" configured to classify an alarm in accordance with one of the following categories: "high", "medium", "low", "actionable/non-actionable advisory" or "no alert".

Another component of the processing unit may be a "top contributor analyser" configured to analyse the priority level determination and to determine the contribution of the feature set contributing to the alarm priority. In other words, the processing unit is configured to identify at least one contributing feature, being a feature of the real-time medical device data or the historical medical data different from the triggering feature, contributing to the determination of the priority level.

The output unit of the device then provides information about the identified triggering feature, the determined priority level, and the at least one identified contributing feature (as the alarm priority data) to a rendering unit, for example.

The rendering unit may be a user interface. Based on the alarm priority data the rendering unit renders/presents the priority level along with the triggering feature and the at least one contributing feature to a user. In other words, the user interface may present the output of the processing unit and display the triggering feature, the alarm priority of the alarm situation and at least one contributor, e.g. active alarms of several medical devices, main vital signs, medication and time stamp etc. That is, in the present invention, by providing at least one contributing feature contributing to the determination of the priority level (and being different from the triggering feature), caregivers are informed about the context of an alarm. In other words, clinicians are informed about the cause of the alarm (triggering feature) and the rationale (contributing feature) behind its assigned priority level. To illustrate the idea, the following examples are given:
A patient with normal vital signs with no recent alarm history has an SpO2 sensor falling off his finger. Usually, if at all, only the reason itself for the alarming situation, i.e. the triggering feature, is presented to the user, i.e. he/she is solely provided with the information that the SpO2 sensor has no signal anymore. However, no further context is provided as to why the alarming situation has a low priority level. In the present invention, further context (at least one contributing feature contributing to the determination of the priority level) is provided, informing the user (e.g. clinician, caregiver etc.) that the vital signs of the patient are normal.

In another example, a patient had an oxygen desaturation 30 minutes ago and the SpO2 sensor has fallen off his finger. Using usual alarm systems, the same alarm would be triggered as in the before mentioned case. However, using the device of the present invention, the alarm priority level would be higher and there would be context information provided explaining the reason (i.e. contributing feature) for the higher priority level, namely the fact that there was a desaturation 30 minutes ago.

In yet another example, the patient is currently having a desaturation and the SpO2 sensor falls off his finger. In this case, using the present invention, the alarm priority level would not be low as usual but high, and the user would be informed about the contributing feature contributing to the determination of this priority level, i.e. the current oxygen desaturation of the patient.

Hence, in the present invention, the alarm priority is determined not only based on an event (rules) that trigger the alarm but also by processing patient context information. This way, alarm prioritization is improved. Caregivers are provided with more accurate alarm priorities of the alarming situation by combining information from alarms and, for example, vital signs (current and past).

Furthermore, alarm burden on caregivers is reduced. The solution provides just one alarm with context to the alarming situation. In existing solutions, caregivers often have to analyse several active alarms and look into patient vital signs to make a clinical decision. By providing at least one contributing feature contributing to the determination of the priority level for the alarming situation, the invention allows caregivers to get information about the context of the alarming situation.

By combining information from alarms and their contributing feature(s) (e.g. vital signs (current and past)) "smart alarming" is created, which can decrease false alarms and in turn decrease alarm fatigue.

In an embodiment of the device, the historical medical data are obtained from at least one medical device and/or a database, particularly a database comprising an electronic health record of the patient.

The database may particularly be a database of a hospital, particularly an Electronic Health Record (EHR) system of a hospital.

For instance, a patient monitor (as medical device) may provide live (i.e. real-time) ECG and SpO2 data, while a hospital EHR database supplies data about the patient's previous cardiac episodes. Both the real-time medical device data and the historical medical data may be used by the presented device. By merging these data the processing unit may be configured to detect that a mild desaturation in a patient and determine a priority level which may warrant higher attention if a history of arrhythmias is contributing to the priority level (compared to a healthy individual). Accordingly, this integration of data enhances context-awareness and enables more accurate alarm prioritization.

In an embodiment of the device, the alarm triggering event is indicative of an exceedance of an alarm limit of a vital sign or of a malfunction of a medical device.

For example, an alarm may be triggered when a patient's heart rate exceeds a predefined threshold, indicating tachycardia, or when a sensor fails to transmit data due to a disconnection or hardware malfunction. In such cases, the device identifies the specific condition and generates an alarm event accordingly.

In another embodiment, the real-time medical device data comprise real-time monitoring data, particularly real-time vital signs data, of the patient, real-time treatment data of the patient and/or real-time malfunction data comprise information about real-time malfunction and/or reduced reliability of the medical device, and/or the historical medical device data comprise information related to prior monitoring and/or treatment of the patient and/or to prior malfunction of the medical device.

Monitoring data of the patient may comprise information about any one of vital signs (e.g. heart rate, blood pressure, respiratory rate, body temperature, oxygen saturation etc.), laboratory test results (e.g. complete blood count, blood glucose level, electrolyte panels etc.), neurological monitoring (e.g. electroencephalography), respiratory monitoring (e.g. pulse oximetry), imaging data (e.g. X-Ray data, computed tomography scans, magnetic resonance imaging etc.). Treatment data of the patient may comprise information about medication dosage, medication times, physiotherapy, radiotherapy etc. Malfunction data comprise information about failures/irregularities that can compromise device performance or patient safety, e.g. information about software errors, hardware malfunctions, user interface problems, calibration errors, delayed responses, complete device failure etc.

In another embodiment, the processing unit is configured to identify a highest contributing feature, the highest contributing feature being a feature of the real-time medical device data or the historical medical data different from the triggering feature contributing to the determination of the priority level with highest significance, wherein the alarm priority data further comprises information about the identified highest contributing feature.

For example, in an alarm scenario, the triggering feature may be an oxygen saturation drop, while the highest contributing feature may be represented by an increased heart rate or a low infusion pump rate. Presenting particularly the highest contributing feature helps caregivers make faster and more informed decisions, particularly in ambiguous clinical situations. In other words, by providing information about the highest contributing feature (with the alarm priority data) clinicians are provided with more context, enhancing clinical decision making.

In another embodiment the processing unit is configured to label (the relevant) features obtained from the real-time medical device data and/or the historical medical data with a significance label indicative of the significance of the feature for the priority level of the alarm , and to identify the highest contributing feature based on the significance labels of the relevant features.

During a desaturation episode, e.g. SpO2 trend, pulse rate variability, and alarm frequency can be extracted as relevant features (contributing to the priority level determined). These features are then labelled with a significance label. For example, for a patient who reacts very negatively to irregularities in his/her pulse the pulse rate variability may be labelled with a significance label corresponding to a higher priority level than SpO2 trend. Based on the significance labels the most impactful/contributing feature(s) can be identified by the processing unit. This structured analysis enhances explainability and aligns alarm decisions with clinical reasoning.

In a further embodiment, the processing unit is configured to apply a machine learning model to the real-time medical device data and the historical medica data for the determination (of the alarm priority level) and identification (of the at least one contributing feature).

Using a machine learning model the determination and identification can be provided in a fast and reliable manner.

In an embodiment, the machine learning model comprises a random forest classifier for labelling the features with the significance label.

In general, there are many ways to solve a classification/labelling problem using machine learning. The random forest classifier has the advantage of being robust to overfitting, especially when dealing with high-dimensional datasets, and can naturally handle both numerical and categorical data. It aggregates determinations from multiple decision trees, thereby increasing generalization performance, and offers intrinsic feature importance metrics, which are useful for identifying the most influential/significant features contributing to alarm prioritization. Moreover, it is relatively fast to train and easy to interpret compared to more complex models like neural networks.

Instead of having one random forest classifier with multi-class outputs (e.g. "high", "medium", "low", "advisory", "no alert"), a combination of multiple random forest classifiers may be used. In this case separate classifiers are trained for each class or class group. For example, a first random forest classifier could distinguish between "high" alarm priority level and all other priority levels, a second random forest classifier could distinguish between "medium" alarm priority level and all other priority levels, a third random forest classifier could distinguish between "low" and all other priority levels and so on. Preferably, for each of the multiple classifiers an optimal/tailored feature set of the real-time medical device data and historical medical data is predetermined and used as input, i.e. for the first random forest classifier distinguishing between high alarm priority level and the other priority levels, a "high feature set" of the real-time medical device data and historical medical data could be used as input, whereas for the second random forest classifier distinguishing between medium alarm priority level and the other priority levels a "medium feature set" of the real-time medical device data and the historical medical data could be used as input.

Using multiple random forest classifiers, the multi-class problem is essentially decomposed into multiple binary classification tasks. This can lead to better performance and allows independent training of data on separate datasets and features.

In yet another embodiment, the processing unit is configured to identify the highest contributing feature based on an explainable artificial intelligence technique, particularly SHapley Additive exPlanations (SHAP).

SHAP is a game theoretic approach to explain the output of any machine learning model. It connects optimal credit allocation with local explanations using the classic Shapley values from game theory and their related extensions. SHAP values provide clinicians with a transparent rationale for the alarm's assigned alarm priority level. In fact, the Shapley value of a feature quantifies how much that feature contributes to shifting the determination from a baseline (e.g., average determination) to the actual outcome (of the alarm priority level). In particular, the SHAP may list the (top) contributors in connection with mathematical content / numerical values, which will need to be passed through a "human readable data converter" so that content could be easily understandable by the end user.

Generally, the machine learning model may be trained based on ground truth data, the ground truth data relating to any one of monitoring of said or another patient, treatment of said or another patient, and a malfunction of said or another medical device, and comprising ground truth data features labelled with a ground truth significance label.

Data collected from intensive care unit events or other treatment episodes, for example, can be collected and annotated by expert clinicians to label alarms as high, medium, or advisory. This expert-based ground truth provides high-quality training data for the model, ensuring that future determinations of the priority level align with real-world clinical expectations. In fact, ground truth generation is an important step for creating a (supervised) machine learning model i.e. collecting data, labelling data and creating features.

To be more precise, data from various medical devices, e.g. patient monitors, ventilators, infusion pumps etc. may be collected using e.g. a standard protocol like SDC ("Service-oriented Device Connectivity") and stored for later processing or directly be processed and fed into a labelling tool. The labelling tool may be represented by the processing unit of the device presented herein or may be represented by a separate tool. In an example scenario, the data collected comprises as features one or more of active alarms for a given timestamp, active wave data for a given timestamp, active vital signs for a given timestamp, historical medical data (patient data, alarm history data, trend data, infusion pump data etc). For a selected time period, the user can decide if an actionable or non-actionable alarming situation is present. For an actionable alarming situation the user can label the alarming situation as "high, medium, low or advisory". For a non-actionable alarming situation the user can label the alarming situation as "advisory" or "no alert". A decision table can be placed for the labelling user to support them in explaining their decision and for guidance from alarm standards.

In the next step, a feature set is generated from the labelled data. For the alarm prioritization classification problem, a first feature would intuitively be the active alarm information and the duration / frequency they have been present. In addition to this, (time series of) vital signs data, infusion pump data etc. could be regarded as features.

In general, a process needs to be determined to find the most useful features from the initial feature set. An example of such process is e.g. Recursive Feature Elimination (RFE), which may help reduce model complexity and performance.

The labeling step creates the ground truth that the machine learning model will learn from. Once the ground truth data is available and the features are (extracted and) labelled, a classification model such as the random forest classifier can be trained (and validated) to solve the alarm prioritization classification problem.

Furthermore, a method can be selected to evaluate the model classification performance with a known metric (e.g. F 1-score). In any case, after training, new data, particularly real-time and historical medical device data, can be processed by the machine learning model to determine the alarm priority level.

In an embodiment, the processing unit is configured obtain the real-time medical device data over a predefined period of time and to escalate or de-escalate the determined priority level based on changes in the real-time medical device data over the predefined period of time.

A change in the state of the alarm priority with time implies an alarm escalation or de-escalation.

If CO2 tubing remains occluded for 60s, the processing unit may determine the alarm priority level to be "advisory". For an occlusion more than 60s (but less than 2 min) the alarm priority level may be determined to be "low", for more than 2 min (but less than 5 min) "medium", for more than 5 min "high". Hence, if CO2 tubing remains occluded for more than 5 minutes despite initial low concern, the alarm priority level may be escalated. Conversely, a resolved desaturation episode may lead to de-escalation of the alarm priority level. This dynamic adaptability improves responsiveness while reducing nuisance alarms. The automatic escalation or de-escalation is important for clinicians to quickly react to the urgency of the situation.

In a further embodiment, the processing unit is further configured to determine, based on the at least one contributing feature, a description related to the at least one contributing feature and/or a corrective measure for reduction of the priority level, wherein the alarm priority data further comprises information about the description and/or the corrective measure.

If low infusion rate is identified as the main contributor to the priority level, a UI of the system may display: "Infusion rate below threshold for 8 minutes; consider increasing rate." This proactive guidance supports prompt, targeted interventions.

In another embodiment, the input unit is further configured to obtain real-time availability data comprising information about real-time availability or location of a clinician and/or clinical equipment, wherein the processing unit is configured to determine the description and/or the corrective measure further based on the real-time availability data.

The provision of additional context information (description / corrective measure) may increase situational awareness and response efficiency.

For example, if it is recognized that a responsible clinician is busy in a distant location, the description related to the highest contributing feature and the corrective measure suggested can be more detailed. Such details can include information about the patient condition prior to the alarm, the current patient condition, predicted, future patient condition changes, an optimal and maximum response time window taking into account the clinician location and patient predicted changes in the patient condition. Using the additional information, the clinician can plan and adapt their reactions more appropriately.

Similarly, for example, if it is expected that an ultrasound device may be needed for examining the patient (based on the observed trend in how the vital signs change), when a low alarm priority level is determined, the description can include the location of the ultrasound, and suggestion to charge it. That way, in case the patient situation deteriorates, and ultrasound is needed the device can be located faster and can be used immediately. If the current clinician is not comfortable with the ultrasound, an expert clinician information can be also included in the alarm description.

Another example could be that more or new medicine is needed for the patient. There could be a scenario where an alarm is triggered and the context description could inform where the medicine is stored and how much is available. The same could also apply for a broken sensor, for example.

### BRIEF DESCRIPTION OF THE DRAWINGS

These and other aspects of the invention will be apparent from and elucidated with reference to the embodiment(s) described hereinafter. In the following drawings:
Fig. 1 shows a schematic diagram of an embodiment of system for improved prioritization of patient-related medical alarms according to the present invention;
Fig. 2 shows a schematic diagram of an embodiment of a device for improved prioritization of patient-related medical alarms according to the present invention;
Fig. 3 shows a flow chart of a first embodiment of a method for improved prioritization of patient-related medical alarms according to the present invention;
Fig. 4 shows a flow chart of a second embodiment of a method for improved prioritization of patient-related medical alarms according to the present invention;
Fig. 5 shows a schematic diagram illustrating a conceptual overview of the device, system and method according to the present invention;
Fig. 6 shows a schematic diagram of a conceptual overview of the processing of the device according to the present invention;
Fig. 7 shows a schematic diagram of a conceptual overview of a method for training a machine learning model which may be applied by the processing unit of the device according to the present invention;
Fig. 8 shows an embodiment of a display of a labelling tool; and
Fig. 9 shows a list of possible features and their description as extracted from labelled medical device data in the process of training a machine learning model which may be applied by the processing unit of the device according to the present invention.

### DETAILED DESCRIPTION OF EMBODIMENTS

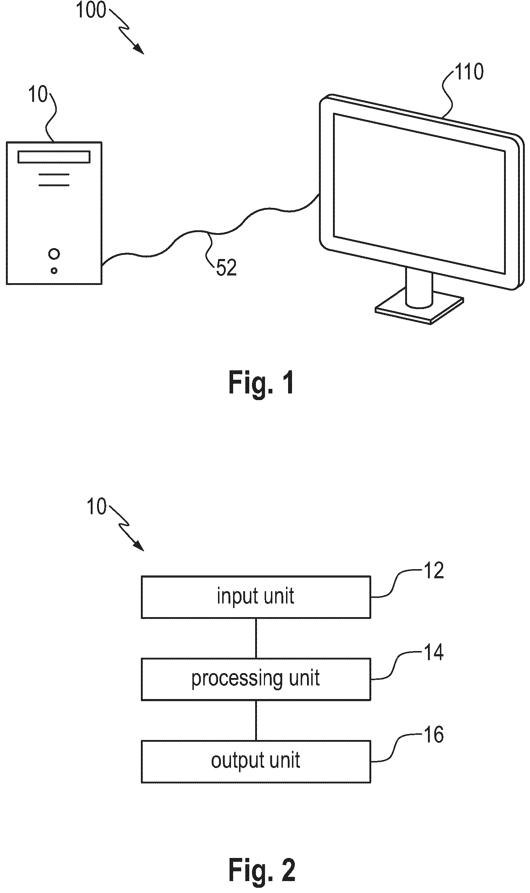

Fig. 1 shows a schematic diagram of an embodiment of system for improved prioritization of patient-related medical alarms according to the present invention.

The system 100 comprises a device 10 for improved prioritization of patient-related medical alarms and a rendering unit 110. The device 10 is configured to obtain (retrieve or receive) (in real time) real-time medical device data of the patient from at least one medical device used for monitoring or treating the patient. Furthermore, the device 10 is configured to obtain historical medical data from a database, for example.

Based on the real-time medical device data the device 10 is configured to determine whether an alarm is to be generated and, if it is determined that an alarm is to be generated, to identify a triggering feature, being a feature of the real-time medical device data being indicative of an event triggering an alarm. The device 10 is further configured to determine, based on real time-medical device data and/or the historical medical data, an alarm priority level indicating a priority of the alarm. Furthermore, the device 10 is configured to identify a at least one contributing feature, the at least one contributing feature being a feature of the real-time medical device data or the historical medical data contributing to (the determination of) the priority level, wherein the at least one contributing feature is different from the triggering feature. Information about the determined priority level and the at least one identified contributing feature is then provided in the form of alarm priority data to the rendering unit 110.

In this embodiment of the system 100, the device 10 and the rendering unit 110 are connected in a wired manner via a cable 52. However, a wireless connection is likewise conceivable.

The rendering unit 110 is configured to render the alarm priority data as visual information. Accordingly, in this embodiment, the rendering unit 110 is implemented as a display.

In general, the rendering unit 110 may be any means that outputs the alarm priority data in visual and/or audible form, e.g. in text form, as image or diagram, as sound or spoken words, etc. For instance, the rendering unit 110 may be a display, a loudspeaker, a touchscreen, a computer monitor, the screen of a smartphone or tablet, etc.

Fig. 2 shows a schematic diagram of an embodiment of a device for improved prioritization of patient-related medical alarms. The device 10 comprises an input unit 12 configured to obtain real-time medical device data, i.e. medical device data which are obtained in real-time from at least one medical device, and historical medical data of the patient (comprising information about monitoring and/or about a malfunction of a medical device used for monitoring/treatment of the patient). The input unit 12 may be directly coupled or connected to a medical device for obtaining (i.e. retrieving or receiving) the real-time medical device data. Similarly, the input unit 12 may obtain the historical medical data from a storage, buffer, network, or bus, etc. The input unit 12 may thus e.g. be a (wired or wireless) communication interface or data interface, such as a Bluetooth interface, WiFi interface, LAN interface, HDMI interface, direct cable connect, or any other suitable interface allowing signal transfer to the device 10.

The processing unit 14 is configured to analyze the obtained real-time medical device data to identify a triggering feature which is indicative of an alarm triggering event. The priority level of the alarm is determined based on the real-time medical device data and/or based on the historical medical data. Besides, the processing unit 14 is configured to find out which feature(s) of the real-time medical device data or the historical medical data have contributed to the determination of the priority level. The processing unit 14 may be any kind of means configured to process the data obtained. It may be implemented in software and/or hardware, e.g. as a programmed processor or computer or app on a user device such as a smartphone, smartwatch, tablet, laptop, PC, workstation, etc.

Information about the contributing feature(s) and the priority level is then output by the output unit 16 of the device, particularly to a rendering unit 110 configured to render said information (alarm priority data). The output unit 16 may generally be any interface configured to provide the alarm priority data, e.g. transmit it to another device or provide it for retrieval by another device (e.g. a smartphone, computer, tablet, etc.). It may thus generally be any (wired or wireless) communication or data interface.

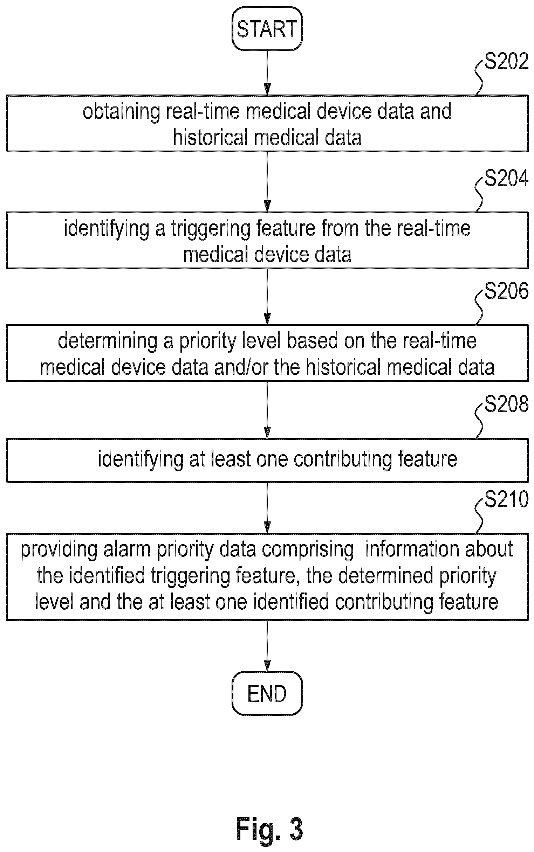

Fig. 3 shows a flow chart of a first embodiment of a method for improved prioritization of patient-related medical alarms according to the present invention.

In a first step S202 of the method real-time medical device data obtained in real-time from at least one medical device and historical medical data are obtained. From on the real-time medical device data a triggering feature, being a feature indicative of an event triggering an alarm, is identified / extracted in step S204.In step S206 a priority level for the alarm of the alarm triggering event is determined based on the obtained real-time medical device data and/or the historical medical data. The priority level may be divided into the four categories high, medium low and advisory, for example. Other levels are likewise conceivable. In step S208 at least one feature contributing to the priority level determined is identified, wherein the contributing feature is different from the triggering feature. In the last step, S210, alarm priority data comprising information about both the priority level and the reason for said level, i.e. the at least one contributing feature, are provided/output.

Hence, using said method a context-specific alarm priority is generated.

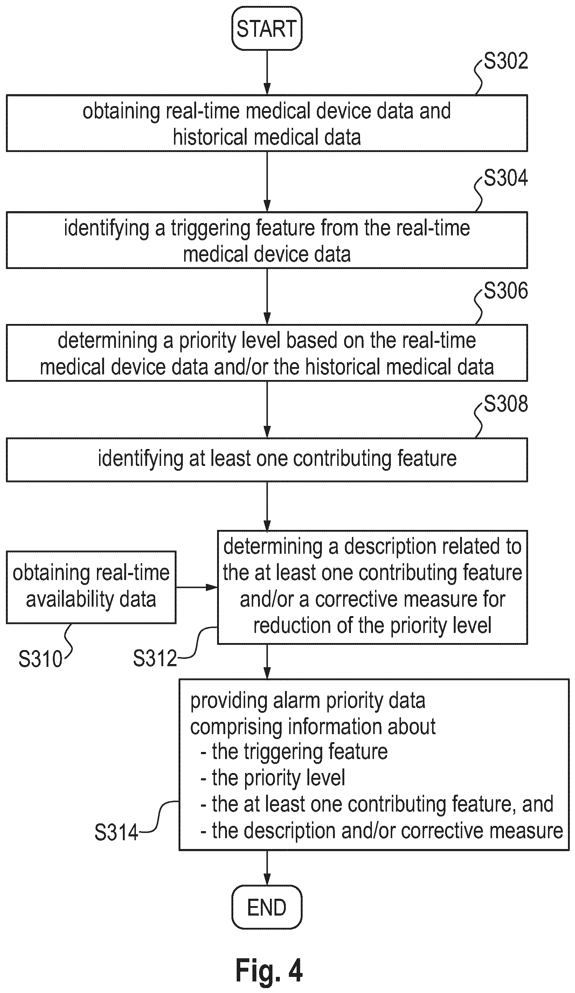

Fig. 4 shows a flow chart of a second embodiment of a method for improved prioritization of patient-related medical alarms according to the present invention. Steps S302 to S308 are identical with steps S202 to S208 of the first embodiment presented. However, contrary to the first embodiment, in the second embodiment of the method there furthermore exists a step S310 of obtaining real-time availability data. These data comprise information about real-time availability or location of a clinician and/or clinical equipment. For example, said data comprises information about the location of the nearest nurse or the availability of an ultrasound examination device. Based on these additionally obtained data, the determined priority level and the at least one identified contributing feature, in step S312, a description related to the contributing feature / the priority level and/or a corrective measure for reduction of the priority level is determined. Said description and/or corrective measure then becomes part of the alarm priority data provided in step S314.

This will help to locate, prepare, and use clinical equipment faster. The same applies to clinical staff, who can be deployed in a more targeted manner. The context information of available clinicians and equipment can be taken into account to update the alarm priority, accompanying information and generally the actions the alarm triggers.

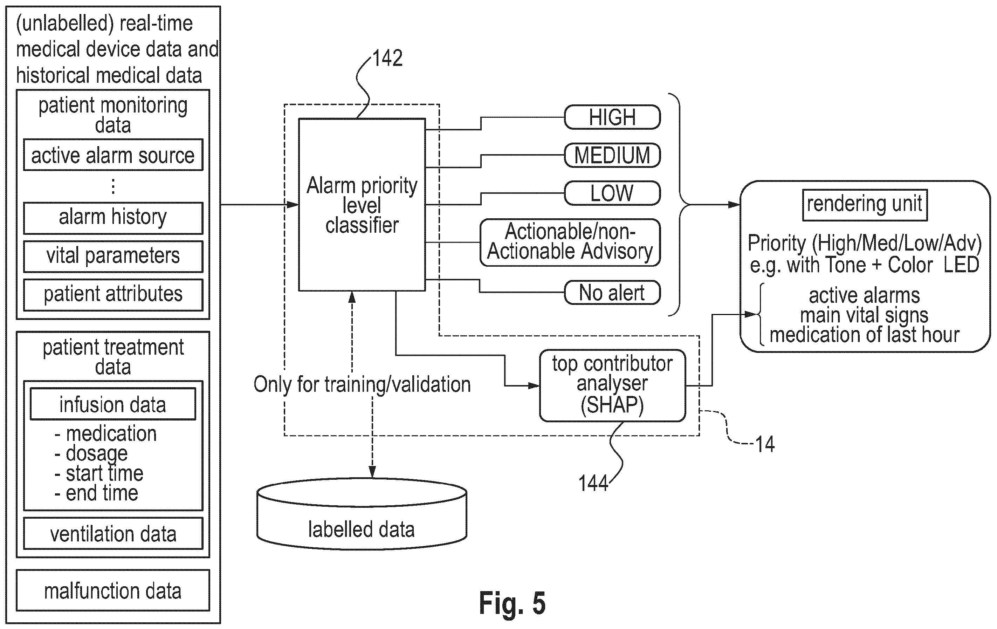

Fig. 5 shows a schematic diagram illustrating a conceptual overview of the device, system and method according to the present invention. On the left, examples of unlabelled real-time medical device data and historical medical data a shown. These data comprise patient monitoring data, e.g. data about vital parameters of the patient or patient attributes, patient treatment data, e.g. infusion data, and malfunction data indication a malfunction of a medical device used for monitoring or treatment of the patient. These data represent the input for the device 10 and may be processed by the processing unit 14 of the device 10 presented herein. Particularly, these data may be processed by an alarm priority classifier (being part of the processing unit 14). The classifier is configured to determine whether the real-time medical device data indicate an alarming situation, i.e. whether an alarm shall be issued, and if it is determined that no alarm shall be issued to set an alarm priority level to "no alert" or if it is determined that an alarm shall be issued to set, based on an analysis of the real-time medical device data and/or the historical medical data, the priority level to "high", "medium", "low" or "actionable/non-actionable advisory" in accordance with the alarming situation recognized. To be more precise, the classifier is part of a machine learning model, the machine learning model being configured to extract relevant features from the obtained data. The relevant features are then labelled/classified by the classifier with a significance label indicating their significance with respect to the (determination of the) priority level. Depending on the (significance) labels of the contributing features the priority level of the alarm is determined.

Besides the processing unit 14 comprises a top contributor analyser which is configured to analyse the determination of the priority level and to determine the contribution of the feature set / most significant feature(s) contributing to the priority level. For this analysis of the contribution of each feature to the final priority level determination, tools like e.g. SHAP are used. Information about the triggering feature and the top contributors along with the priority level are then rendered by the rendering unit 110. For example, said information may be presented on a display.

As can also be seen from Fig. 5 the alarm priority level classifier is trained using labelled data, particularly labelled real-time medical device data and (historical) medical data (from the same or another patient).

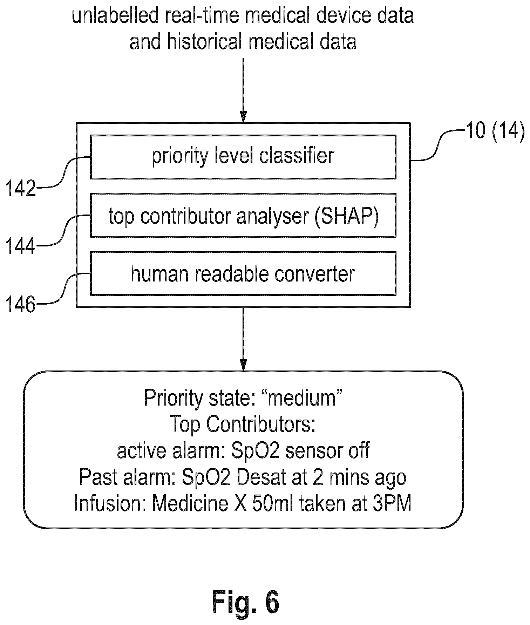

Fig. 6 shows a schematic diagram of a conceptual overview of the processing of the device 10. Unlabelled real-time medical device data and historical medical data are first analysed by the priority level classifier 142 of the processing unit 14 to determine a priority level corresponding to said data. A top contributor analyser 144 using SHAP is then used for finding an "explanation" for the priority level determined, i.e. to find the highest contributing features. As the output of the top contributor analyser 144 may comprise information which is not directly readable/understandable by humans, a human readable converter 146 may be used for post-processing, particularly for converting the SHAP output into human readable context. The output of the processing unit 14 (device 10) may then be rendered by a rendering unit 110. In the given example, the determined priority level is determined to be medium, the reason being the top contributing features displayed on a display of the rendering unit 110.

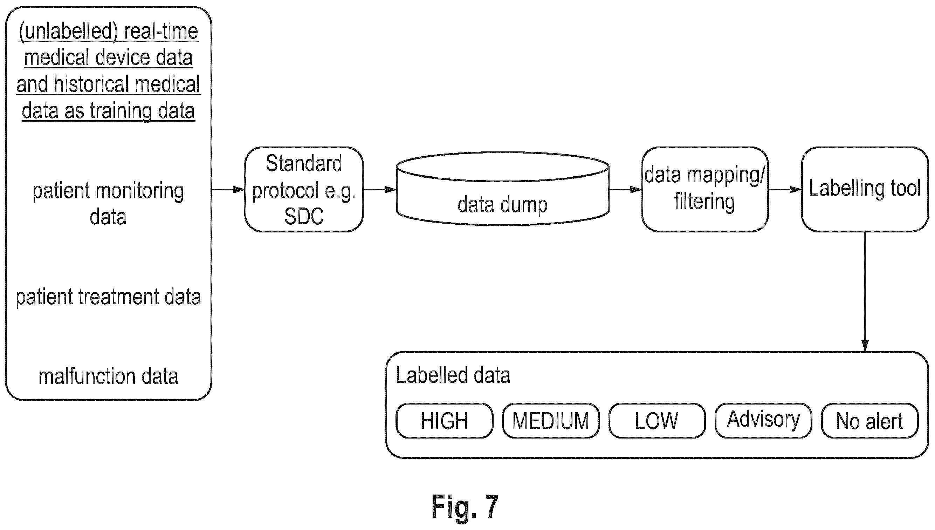

Fig. 7 shows a schematic diagram of a conceptual overview of a method for training a machine learning model which may be applied by the processing unit 14. In a first step unlabelled real-time medical device data and historical medical data are collected using standard protocols like SDC and stored in a data dump / storage. Prior to feeding said data into a labelling tool, the medical device data are preferably be mapped and/or filtered in accordance with the requirements of the labelling tool. In other words, the data may be pre-processed to make them (more) compatible with the labelling tool. This way, it can be ensured that the user experience of the labelling tool remains the same even if a new medical device is introduced. The labelling tool then labels the data with significance labels indicating the significance of the data (particularly their features) for the priority level of the data. Preferably, the levels "high", "medium", "low", "advisory" and "no alert" are used.

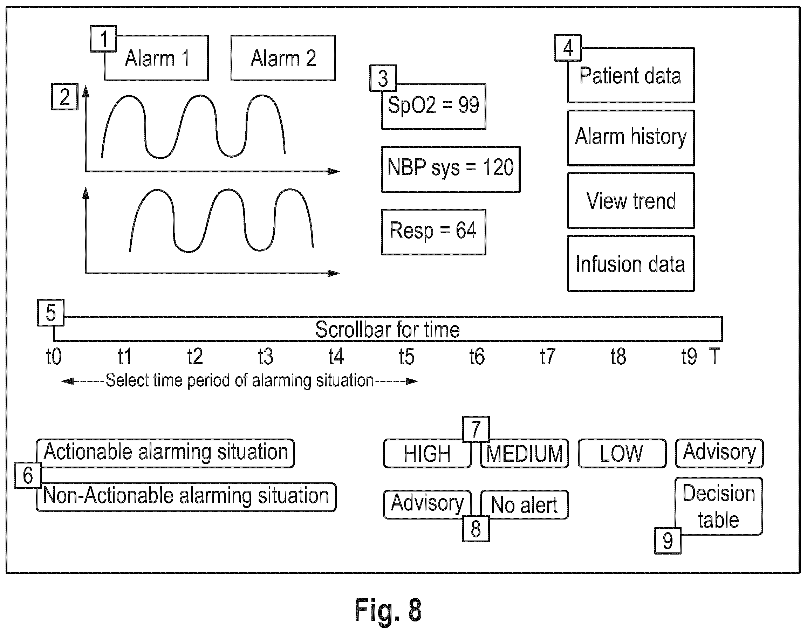

Fig. 8 shows an embodiment of a display of a labelling tool. On the display of the labelling tool the user can see active alarms for a given timestamp (denoted by "1"). active wave data for a given timestamp (denoted by "2"), active vital signs for a given timestamp (denoted by "3"), historical medical data (patient data, alarm history data, trend, infusion pump; denoted by "4"). Furthermore, the tool allows the user to scroll through time and visualizes patient condition with the scrollbar (said feature of the tool being denoted by "5"). Using the scrollbar, the user can select the time period of the alarming situation. For a selected time period, the user can select if the alarmin situation is an actionable or non-actionable alarming situation (the selection possibilities being denoted by "6"). For actionable alarms the user can label the alarming situation as high, medium or low (the corresponding selection being denoted by "7"). For non-actionable alarms the user can label the alarming situation as "advisory" or "no alert" (the corresponding selection being denoted by "8"). A decision table for decision support and guidance are denoted by "9".

Once the real-time medical device data and the historical medical device data are labelled, in the next step of training the machine learning model features are generated from the labelled data. For the alarm prioritization classification problem, a first feature would intuitively be the active alarm information and the duration they have been present. In addition to this, time series of vital signs data, infusion pump data etc. could be extracted as features.

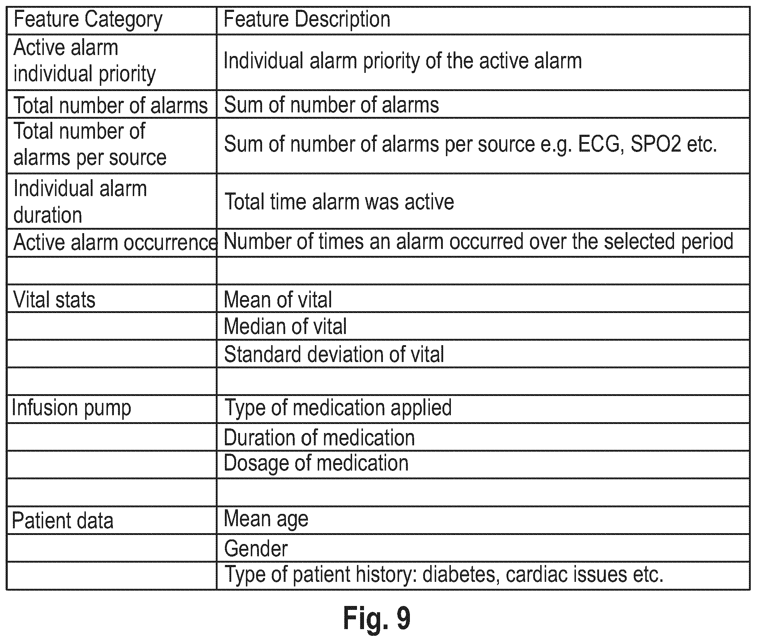

Fig. 9 shows a list of possible features and their description as extracted from labelled real-time medical device data and historical medical data in the process of training a machine learning model which may be applied by the processing unit 14 of the device 10 according to the present invention. The list of the features shown is not complete. In fact, further and/or other features may be extracted.

Once labelling and training is finished, the determination phase can start, i.e. the machine learning model is ready to be applied to medical device data. The trained model can then determine the priorities of alarming situations from unlabelled data. Furthermore, the model can indicate the (top) contributing features for the determination.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (10) for improved prioritization of patient-related medical alarms, the device (10) comprising:
- an input unit (12) configured to obtain real-time medical device data obtained in real-time from at least one medical device and historical medical data of the patient, the real-time medical device data relating to any one of monitoring of the patient and a malfunction of a medical device used for monitoring or treatment of the patient;
- a processing unit (14) configured to:
- identify a triggering feature, being a feature of the real-time medical device data indicative of an event which triggers an alarm;
- determine, based on the real-time medical device data and the historical medical data a priority level indicating a priority of the alarm; and
- identify at least one contributing feature, being a feature of the real-time medical device data and/or the historical medical data different from the triggering feature, contributing to the determination of the priority level; and
- an output unit (16) configured to provide alarm priority data comprising information about the identified triggering feature, the determined priority level, and the at least one identified contributing feature.

2. Device (10) according to claim 1,
wherein the historical medical data are obtained from at least one medical device and/or a database, particularly a database comprising an electronic health record of the patient.

3. Device (10) according to claim 1 or 2,
wherein the alarm triggering event is indicative of an exceedance of an alarm limit of a vital sign or of a malfunction of a medical device.

4. Device (10) according to any one of the preceding claims 1 to 3,
wherein the real-time medical device data comprise real-time monitoring data, particularly real-time vital signs data, of the patient, real-time treatment data of the patient and/or real-time malfunction data comprise information about real-time malfunction and/or reduced reliability of the medical device, and/or wherein the historical medical device data comprise information related to prior monitoring and/or treatment of the patient and/or to prior malfunction of the medical device.

5. Device (10) according to any one of the preceding claims 1 to 4,
wherein the processing unit (14) is configured to identify a highest contributing feature, the highest contributing feature being a feature of the real-time medical device data or the historical medical data different from the triggering feature contributing to the determination of the priority level with highest significance , wherein the alarm priority data further comprises information about the highest contributing feature.

6. Device (10) according to claim 5,
wherein the processing unit (14) is configured to label the relevant features obtained from the real-time medical device data and/or the historical medical data with a significance label indicative of the significance of the feature for the priority level of the alarm and to identify the highest contributing feature based on the significance labels of the relevant features.

7. Device (10) according to any one of the preceding claims 1 to 6,
wherein the processing unit (14) is configured to apply a machine learning model to the real-time medical device data and historical medical data for the determination and identification.

8. Device (10) according to claims 6 and 7,
wherein the machine learning model comprises a random forest classifier for labelling the features with the significance label.

9. Device (10) according to claim 5,
wherein the processing unit (14) is configured to identify the highest contributing feature based on an explainable artificial intelligence technique, particularly SHapley Additive exPlanations.

10. Device (10) according to any one of the preceding claims 1 to 9,
wherein the processing unit (14) is configured to obtain the real-time medical device data over a predefined period of time and to escalate or de-escalate the determined priority level based on changes in the real-time medical device data over the predefined period of time.

11. Device (10) according to claim 6,
wherein the processing unit (14) is further configured to determine, based on the at least one contributing feature, a description related to the at least one contributing feature and/or a corrective measure for reduction of the priority level, wherein the alarm priority data further comprises information about the description and/or the corrective measure.

12. Device (10) according to claim 11,
wherein input unit (12) is further configured to obtain real-time availability data comprising information about real-time availability or location of a clinician and/or clinical equipment, wherein the processing unit (14) is configured to determine the description and/or the corrective measure further based on the real-time availability data.

13. System (100) for improved prioritization of patient-related medical alarms, the system (100) comprising:
- a device (10) as claimed in claim 1; and
- a rendering unit (110) configured to render visual and/or audible information in accordance with the alarm priority data.

14. Method for improved prioritization of patient-related medical alarms, the method comprising:
- obtaining real-time medical device data obtained in real-time from at least one medical device and historical medical data of the patient, the real-time medical device data relating to any one of monitoring of the patient and a malfunction of a medical device used for monitoring or treatment of the patient;
- identifying a triggering feature, being a feature of the real-time medical device data indicative of an event which triggers an alarm;
- determining, based on the real-time medical device data and the historical medical data a priority level indicating a priority of the alarm;
- identifying at least one contributing feature, being a feature of the real-time medical device data and/or the historical medical data different from the triggering feature, contributing to the determination of the priority level; and
- providing alarm priority data comprising information about the identified triggering feature, the determined priority level and the at least one identified contributing feature.

15. Computer program comprising program code means for causing a computer to carry out the steps of the method as claimed in claim 14 when said computer program is carried out on the computer.
